# FASCICULE DE BREVET EUROPEEN

(11) **EP 0 368 702 B1**
(45) Date de publication et mention de la délivrance du brevet: **26.04.1995**
(21) Numéro de dépôt: 89402827.3
(22) Date de dépôt: 13.10.1989
(51) Int. Cl.: C12N 11/08

(54) **Support pour molécules biologiquement actives ou lui-mème biologiquement actif, son procédé de préparation et son application en biologie**
Träger für biologisch wirksame Moleküle oder biologisch wirksamer Träger, Verfahren zu seiner Herstellung und seine biologische Verwendung
Carrier for biologically active molecules, or itself biologically active, process for its preparation and its biological use

(30) Priorité: 21.10.1988 FR 8813798
(43) Date de publication de la demande: 16.05.1990
(73) Titulaire: SOCIETE PROLABO, 94120 Fontenay-sous-Bois (FR)
(72) Inventeur: Vinot, Bernard, F-75010 Paris (FR)
(74) Mandataire: Ahner, Francis

(56) Documents cités:
- FR-A- 2 322 155
- FR-A- 2 476 125
- US-A- 4 071 409
- US-A- 4 218 363
- US-A- 4 268 423
- US-A- 4 337 172

## Description

La présente invention a pour objet un support pour molécules biologiquement actives ou lui-même biologiquement actif, son procédé de préparation et son application en biologie (chromatographie d'affinité, tests de diagnostic, culture de cellules).

Il est connu de fixer des molécules biologiquement actives sur des particules de polymère en dispersion aqueuse par adsorption lorsqu'il s'agit de particules de polystyrène par exemple, ou par covalence lorsqu'il s'agit de polymères présentant des groupes réactifs.

L'avantage présenté par les latex réside dans la grande surface spécifique développée par les particules et dans leur large gamme de fonctions chimiques disponibles. Leur inconvénient, par contre, réside dans la difficulté de les utiliser, une fois atomisés, comme système de garnissage dans des colonnes ou des capillaires ; en effet, lorsque les particules de polymère ont un diamètre inférieur à 1 micron le système de garnissage obtenu manque de perméabilité.

La Demanderesse a trouvé un support pour molécules biologiquement actives ou lui-même biologiquement actif pouvant servir de système de garnissage, ledit système étant tout à la fois perméable et de grande surface spécifique.

Le support pour molécules biologiquement actives faisant l'objet de l'invention, est caractérisé en ce qu'il est constitué :
- d'une matrice inorganique, poreuse, non hydrosoluble
- et, à la surface de ladite matrice, de particules de polymère non hydrosoluble susceptibles de fixer des molécules biologiquement actives.

Le terme "surface" siginifie aussi bien la surface externe de la matrice que sa surface interne, c'est-à-dire celle développée par les pores.

Parmi les matériaux inorganiques poreux, non hydrosolubles, pouvant constituer la matrice, on peut citer les silices, les alumines, les zéolites, la brique, les oxydes de titane, de zirconium, les verres poreux.

Les silices et les alumines sont utilisées à titre préférentiel. Le matériau constituant la matrice peut éventuellement présenter en surface des groupes fonctionnels (-COOH, -NH₂, -OH, -SH, ...) susceptibles de réagir par covalence avec les particules de polymère.

Selon l'invention, la matrice peut présenter un volume poreux de l'ordre de 0,5 à 1,8 ml/g, un diamètre de pores de l'ordre de 0,005 à 10 microns (de préférence de 0,1 à 1 micron) et une surface spécifique de l'ordre de 2 à 1000 m²/g.

Elle se présente de préférence sous forme de particules, de billes notamment, présentant un diamètre de l'ordre de 4 microns à 5 mm (de préférence de 50 à 500 microns).

On entend par "monomère non miscible à l'eau" les monomères présentant une solubilité dans l'eau inférieure à 5 % en poids.

Parmi ceux-ci on peut citer :
- les monomères vinylaromatiques (styrène, vi- nyltoluène ...)
- les alkylesters d' acides α-e insaturés (acry- lates et méthacrylates de méthyle, éthyle ...)
- les esters d'acides carboxyliques insaturés (acétate de vinyle
- le chlorure de vinyle ; le chlorure de vinylidène
- les diènes (butadiène ...)
- ceux présentant des fonctions nitriles (acrylo- nitrile ...)
- les siloxanes.

La composition monomère dont dérive ledit polymère peut contenir en outre jusqu'à 10 % de son poids (de préférence jusqu'à 4 % de son poids) d'au moins un monomère portant des groupes ionogènes ou réactifs tels que :- S03H, OSOsH, - NR₃, - COOH, - OH, - NH₂, - NR₂, 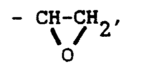 - 0 CH₂CI, - CONH₂, - SH, 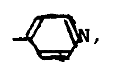 - COOR, - PO(OR)₂,
R représentant un radical alkyle en Ci - C₄, de préférence en Ci - C₂

A titre d'exemple on peut citer :
- le vinylbenzène sulfonate, les sulfoalkylesters d'acides insaturés (2-sulfoéthylméthacrylate ...).
- les acides carboxyliques insaturés (acide acrylique, méthacrylique, maleïque, itaconi- que ...)
- les hydroxyalkylacrylates ou méthacrylates (acrylate d'hydroxyéthyle, hydroxypropyle ...)
- les aminoalkylesters d'acides insaturés (2-aminoéthylméthacrylate ...)
- l'acrylamide
- le chlorure de vinylbenzyle
- le méthacrylate de glycidyle.

Lesdites particules de polymère peuvent présenter une granulométrie de l'ordre de 0,05 à 20 microns et de préférence de l'ordre de 0,1 à 3 microns. D'une manière toute préférentielle le diamètre des particules de polymère est inférieur à celui des pores de la matrice et en particulier inférieur à 1 micron.

Le poids de la matrice correspond à environ 1 à 1 000 fois celui des particules de polymère (de préférence environ 50 à 500 fois).

Ledit support pour molécules biologiquement actives faisant l'objet de l'invention peut être préparé par "dépôt" des particules de polymère à la surface de la matrice par simple contact d'une dispersion aqueuse (latex) de particules de polymère avec la matrice poreuse, puis séchage après lavage éventuel (à l'eau distillée). Le latex mis en oeuvre contient environ 0,1 à 50 % (de préférence de 0,5 à 15 %) en poids de particules de polymère.

Les quantités relatives de matrice et de dispersion aqueuse mises en oeuvre correspondent à un rapport pondéral matrice/particules de polymère de l'ordre de 0,01 à 1 000 ; selon la concentration de la dispersion et les caractéristiques physiques de la matrice, la quantité de particules mise en oeuvre correspond à environ 1 à 100 fois celle déposée.

La dispersion non utile peut ensuite être remise en oeuvre, après séparation du support obtenu, dans une opération ultérieure.

Lorsque ledit support est préparé à partir d'une matrice présentant en surface des groupes fonctionnels susceptibles de réagir par covalence avec les particules de polymère, il est préférable de préactiver la matrice à l'aide d'un agent de couplage (carbodiimide hydrosoluble, glutaraldéhyde, N-hydroxzybenzotriazole, bromure de cyanogène,...) puis de la mettre en contact avec la dispersion de polymère.

Ledit support ci-dessus décrit peut être utilisé en biologie pour immobiliser des substances biologiquement actives (protéines telles que anticorps, enzymes ... ; antigènes ; médicaments...) par adsorption ou covalence ; selon la nature de ladite substance active on peut obtenir un support susceptible d'être avantageusement utilisé dans les tests de diagnostic (agglutination - "RIA" radioimmunological assay - "IRMA" immunoradiometric assay - "EIA" enzyme immuno assay), en chromatographie d'affinité, comme catalyseur enzymatique en biotechnologie, comme support de culture cellulaire.

La présente invention a également pour objet les supports biologiquement actifs constitués :
- d'une matrice inorganique, poreuse non hydrosoluble
- et, à la surface de ladite matrice, de particules de polymère non hydrosoluble "sensibilisé" par une quantité efficace de substances biologiquement actives.

La quantité de substance "sensibilisante" présente est généralement de l'ordre de 0,01 à 15 % en poids par rapport au poids des particules de polymère. Il est bien évident que cette quantité est choisie en fonction de l'application recherchée du support biologiquement actif.

On entend par particules de polymère "sensibilisé" par des substances biologiquement actives, des particules de polymère sur lesquelles lesdites substances sont fixées par adsorption ou covalence.

La nature et les caractéristiques de la matrice ainsi que la nature et les caractéristiques des particules de polymère ont déjà été données ci-dessus.

Parmi les substances biologiquement actives "sensibilisantes" on peut citer les protéines (anticorps, enzymes ...), les antigènes, les médicaments...

Lesdits supports biologiquement actifs faisant l'objet de l'invention peuvent être préparés par "dépôt" des particules de polymère "sensibilisé" par des substances biologiquement actives à la surface de la matrice par simple contact d'une dispersion aqueuse (latex) de particules de polymère "sensibilisé" par des substances biologiquement actives, avec la matrice poreuse, puis séchage après lavage éventuel, à une température compatible avec la substance biologiquement active fixée sur lesdites particules.

La "sensibilisation" des particules de polymère par la substance biologiquement active peut être réalisée par adsorption ou bien par couplage, la réaction de couplage faisant intervenir les groupes superficiels réactifs ou fonctionnels du polymère et les groupements fonctionnels de la molécules biologique à fixer.

La réaction de couplage peut être réalisée selon des méthodes bien connues, par exemple :
. en faisant appel à des agents de couplage (tels que glutaraldehyde, carbodiimide hydrosoluble, N-hydroxybenzotriazole spacers du type 1-6 diaminohexane, polysaccharide...)
. par activation des fonctions du polymère (par exemple par diazotation, par action du bromure de cyanogène, du chlorure de tosyle...)

puis réaction avec la molécule à fixer.
etc...

Des exemples de réactions de couplage sont donnés dans les brevets américains n ° 3.857.931, 4.045.384, 4.140.662, 4.046.723, 4.421.896, anglais n _{°} 2.004.892, français n _{°} 2.331.567, n _{°} 2.345.459, 2.378.094, européen n 15.841 ...

Une variante de réalisation du procédé de préparation desdits supports biologiquement actifs consiste à "déposer" des particules de polymère non "sensibilisé" à la surface de la matrice selon le procédé précédemment décrit concernant les supports pour molécules biologiquement actives, puis à fixer les molécules biologiquement actives sur les particules de polymère par adsorption ou couplage selon les méthodes ci-dessus décrites.

Les supports biologiquement actifs faisant l'objet de l'invention peuvent être utilisés, selon la nature de la substance active immobilisée sur les particules de polymère, dans les tests de diagnostic ("RIA" radioimmunological assay - "IRMA" immunoradiometric assay - "EIA" enzyme immuno assay), en chromatographie d'affinité, comme catalyseur enzymatique en biotechnologie, comme support de culture cellulaire.

Ils sont particulièrement intéressants comme tests de diagnostic lorsque la substance active immobilisée est un immunoréactif (antigène ou anticorps) ; sous forme particulaire lesdits supports peuvent constituer le garnissage de colonnes et en particulier de tubes capillaires utilisables pour la réalisation de tests qualitatifs ou semi-quantitatifs.

Les exemples suivants sont donnés à titre indicatif et ne peuvent être considérés comme un limite du domaine et de l'esprit de l'invention.

### EXEMPLE 1

### Préparation d'un latex de particules de polymère "sensibilisé"

Des fragments d'anticorps F (ab')₂ Lapin anti protéine C sont mis en incubation à la concentration de 1 mg/I dans un tampon phosphate salin à pH 7,8 en présence d'un latex ESTAPOR K 025 commercialisé par RHONE-POULENC (latex calibré constitué de microsphères de polystyrène de 0,25 micron de diamètre) à la concentration de 1 % en poids de particules de polymère dans le tampon.

Après 17 h d'incubation à 22°C le latex sensibilisé est lavé trois fois à l'eau, centrifugé puis remis en dispersion dans un tampon phosphate gélatine à la concentration de 1 % en poids de particules de polymère dans le tampon.

### Dépôt des particules de polymère "sensibilisé" sur une silice poreuse

On y ajoute en pluie fine et sous faible agitation de la silice SPHEROSIL X 005 commercialisé par I B F (grains de silice de 100 à 200 microns de diamètre, présentant un volume poreux de 1 ml/g, un diamètre de pores de 0,30 micron et une surface spécifique de 10 m²/g), à raison de 10 % en poids par rapport au poids de dispersion (soit 1 000 % en poids par rapport au poids des particules).

Après deux heures d'imprégnation au repos et à température ambiante, la silice est lavée cinq fois à l'eau distillée, puis séchée à l'étuve à 50 °C pendant deux heures.

### Confection d'un test capillaire

Un tube capillaire du type pipette de PASTEUR est fermé à son extrémité inférieure par un tampon de coton hydrophile. La partie capillaire du tube est remplie sous vibration à l'aide d'un agitateur du type REAX 2000 commercialisé par Prola- bo, de silice précédemment préparée, sur une hauteur de 5 cm.

L'extrémité supérieure du tube capillaire est fermée d'un tampon de coton hydrophile.

### Test qualitatif de diagnostic (technique du sandwich)

L'extrémité inférieure du tube capillaire est plongée dans une solution aqueuse de protéine C (antigène).

La colonne de silice est remplie en moins de 5 minutes. Le tube est incubé pendant 2 heures à température ambiante. Le tube, muni d'un compte gouttes en aspiration est plongé dans une solution de lavage (tampon phosphate TWEEN) puis renversé pour éliminer l'eau de lavage.

Le tube, muni de son compte gouttes en de- pression est ensuite plongé dans une solution aqueuse d'anticorps antiprotéine C-peroxydase jusqu'à imprégnation totale du support.

Après incubation pendant deux heures à température ambiante puis lavage comme précédemment, une solution de substrat ABTS (à base de 2-2' azinodi-3 éthylbenzthiazoline sulfate) révèle la présence de protéine C par coloration de la silice en bleu- vert.

### EXEMPLE 2

### Réactifs utilisés

- silice : SPHEROSIL X 005
- latex : ESTAPOR K1 - 010 (microsphères de polystyrène carboxylé présentant 443 mi- croeq/g fonctions - COOH et un diamètre de 0,175 micron).
- anticorps : antirabblit Ig G horse radish peroxydase commercialisé par AMERSHAM
- reactif de couplage : 3 - diméthylaminopro- pylcarbodiimide (C D I) commercialisé par ALDRICH
- tampon : MES à pH 5,5 (solution aqueuse à 0,05 M/I de morpholinoethane sulfonique commercialisée par MERCK)
- substrat : ABTS (solution aqueuse à 1 mM/I de 2,2' azinodi-3 ethyl benzthiazoline sulfate)
- stabilisant : solution aqueuse à 0,1 M/I d'acide citrique et à 0,01 % en poids de N. N₃

### Dépôt du polymère sur la silice

Le latex à la concentration initiale du 10 % en poids est dilué par de l'eau distillée jusqu'à une concentration de 0,5 % en poids.

On ajoute au latex dilué 10 % en poids de silice (par rapport au latex dilué) ; cet ajout se fait en pluie fine en 10 secondes sous ultrasons.

Le mélange obtenu est placé sur un rouleau tournant à faible vitesse (environ 1 rpm) durant 3 heures, puis stocké à température ambiante pendant 1 nuit.

Le produit obtenu est lavé par 5 fois 100 ml d'eau distillée par gramme de silice, puis séché à 45 _{°} C pendant 24 heures.

### Sensibilisation

1 g du produit préparé ci-dessus est introduit dans 10 ml d'une solution à 1 mg/ml de réactif de couplage CDI dans l'eau distillée.

Après incubation pendant 30 minutes à 50 °C, le produit obtenu est lavé par 3 fois 10 ml de tampon MES puis mis en contact avec 5 ml d'une solution contenant 10 mg/ml d'anticorps peroxydase dans le tampon MES.

Après incubation pendant 90 minutes à 37°C on lave le support bioligiquement actif ainsi obtenu par 3 fois 20 ml de tampon MES. On obtient ainsi une suspension de support biologiquement actif.

### Dosage

L'anticorps fixé est dosé par mesure de densité optique sur spectrophotomètre ELISA Minireader Il (commercialisé par DYNATECH).

L'appareil est étalonné comme suit avec des solutions d'anticorps peroxydase en concentration décroissante (allant de 0,25 mg/ml à 0,03 mg/ml) dans le tampon MES.

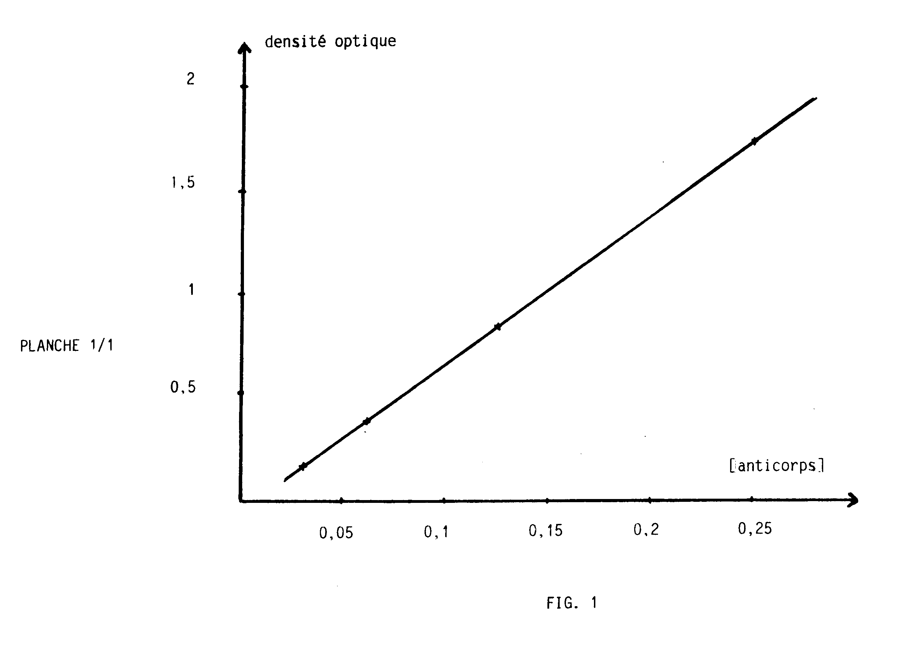

100 microlitres des solutions d'anticorps peroxydase sont placés dans les puits de la microplaque ELISA et additionnés de 100 microlitres de solution de ABTS. Après 10 minutes d'incubation à température ambiante, on introduit dans chaque puits 50 microlitres d'une solution aqueuse à 0,1 M d'acide citrique et à 0,01 % en poids de Na₃N, pour stopper la réaction, avant de mesurer les densités optiques ; la droite d'étalonnage est représentée à la figure 1.

Une mesure de densité optique est réalisée dans les mêmes conditions sur le surnageant de la suspension de support biologiquement actif obtenu à l'opération de sensibilisation. La valeur obtenue est de l'ordre de 0,15, ce qui correspond d'après la figure 1 à une quantité d'anticorps libres inférieure à 0,03 mg/ml (dont on tiendra compte dans le calcul du nombre d'anticorps fixés sur le support).

On prélève un échantillon de 0,5 ml de suspension de support biologiquement actif préparée à l'opération de sensibilisation, échantillon que l'on place dans un tuble taré.

On y ajoute 0,5 ml de solution d'ABTS ; le mélange est incubé pendant 10 minutes à température ambiante ; le produit libéré par la réaction de l'anticorps fixé sur le support et l'ABTS passe en solution. 200 microlitres de surnageant sont alors placés dans un puits de la microplaque ELISA avec 50 microlitres de solution aqueuse à 0,1 M d'acide citrique et à 0,01 % en poids de N. N₃.

La densité optique corrigée (après avoir retranché 0,15) est de 1,52 ce que correspond d'après la figure 1 à 0,217 mg/ml d'anticorps, soit 0,05425 mg d'anticorps pour 250 microlitres de suspension (200 microlitres de surnageant + 50 microlitres de solution stabilisante).

Le support est séparé du milieu liquide puis placé à l'étuve à 80°C pendant 24 heures et pesé.

Sa masse est de 0,03155 g.

La quantité d'anticorps fixée par gamme de silice est donc 0,05425/0,03155 = 1,719 mg/g.

### EXEMPLE 3

On répète l'opération décrite à l'exemple 2 dans les mêmes condition à partir de :
- latex ESTAPOR Ai -005 (microsphères de copolymère styrène - acrylate présentant 444 mi- croeq/g fonctions - COOH et un diamètre de 0,07 micron).

la densité optique corrigée est de 1,65, ce que correspond à 0,237 mg/ml d'anticorps, soit 0,005925 mg d'anticorps pour 250 microlitres de suspension. La masse du support et de 0,01965 g. La quantité d'anticorps fixée par gramme de silice est de 3,015 mg/g.

### EXEMPLE 4

### Préparation du support

On ajoute en pluie fine es sous faible agitation du verre poreux C.P.G.^{R} (commercialisé par Controlled Pore Glass, présentant un diamètre de pores de 0,14 micron, une surface spécifique de 13 m²/g, un volume poreux de 0,7 ml/g et un diamètre de particule de 125 microns) à un latex ESTAPOR A₁ -005, à savoir de 10 % en poids par rapport au poids de dispersion.

Après 2 heures d'imprégnation au repos et à température ambiante, le support obtenu est lavé cinq fois à l'eau distillée Sensibilisation

Des fragments d'anticorps F (a b')₂ Lapin anti proteine C sont mis en incubation à la concentration de 1 mg/I dans un tampon phosphate salin à pH 7,8 en présence de la suspension de support préparée ci-dessus, à la concentration de 1 % poids de support sec dans le tampon.

Après 17 heures d'incubation à 22 _{°} C le support sensibilisé obtenu est lavé trois fois à l'eau, puis séparé du milieu liquide et séché à l'étuve à 50 _{°} C pendant 2 heures.

Un test capillaire peut être confectionné selon le processus de l'exemple 1.

## Revendications (Revendications pour l'(les) Etat(s) contractant(s) suivant(s) : suivants : AT, BE, CH, LI, DE, FR, GB, IT, LU, NL, SE)

1. Support pour molécules biologiquement actives caractérisé en ce qu'il est constitué :
- d'une matrice inorganique, poreuse, non hydrosoluble,
- et à la surface de ladite matrice, de particules de polymère non hydrosoluble susceptibles de fixer des molécules biologiquement actives.

2. Support selon la revendication 1, caractérisé en ce que ladite matrice présente un volume poreux l'ordre de 0,5 à 1,8 ml/g, un diamètre de pores de l'ordre de 0,005 à 10 microns et une surface spécifique de l'ordre de 2 à 1 000_{m2/}g.

3. Support selon la revendication 1 ou 2, caractérisé en ce que ladite matrice se présente sous forme de particules de diamètre de l'ordre de 4 microns à 5 mm.

4. Support selon l'une quelconque des revendications précédentes, caractérisé en ce que la matière constituant la matrice est de la silice, de l'alumine, du verre poreux.

5. Support selon l'une quelconque des revendications précédentes, caractérisé en ce que les particules de polymère présentent une granulométrie de l'ordre de 0,05 à 20 microns.

6. Support selon la revendication 5, caractérisé en ce que le diamètre des particules de polymère est inférieur à celui des pores de la matrice.

7. Support selon l'une quelconque des revendications précédentes, caractérisé en ce que le poids de la matrice correspond à environ 1 à 1 000 fois celui des particules de polymère.

8. Support selon l'une quelconque des revendications précédentes, caractérisé en ce que le polymère non hydrosoluble dérive d'un monomère vinylaromatique, d'un alkylester d'acide a-z insaturé, d'un ester d'un acide carboxylique insaturé, du chlorure de vinyle, du chlorure de vinylidène, d'un diène conjugué, d'un monomère insaturé présentant une fonction nitrile, d'un siloxane.

9. Support selon l'une quelconque des revendications précédentes, caractérisé en ce que le polymère non hydrosoluble dérive d'un monomère non miscible à l'eau et de moins de 10 % d'au moins un comonomère portant un groupe ionogène ou réactif.

10. Support selon la revendication 9, caractérisé en ce que le comonomère est du vinylbenzène sulfonate, un sulfoalkylester d'acide insaturé, un acide carboxylique insaturé, un hydroxyal- kylacrylate ou méthacrylate, un aminoalkyles- ter d'acide insaturé, l'acrylamide, le chlorure de vinylbenzène, le méthacrylate de glycidyle.

11. Support selon l'une quelconque des revendications précédentes, caractérisé en ce que la matrice présente en surface des groupes fonctionnels susceptibles de réagir par covalence avec les particules de polymère.

12. Procédé de préparation du support faisant l'objet de l'une quelconque des revendications précédentes par dépôt de particules de polymère non hydrosoluble susceptibles de fixer des molécules biologiquement actives à la surface d'une matrice inorganique, poreuse, non hydrosoluble, par contact d'une dispersion aqueuse desdites particules de polymère avec la matrice poreuse et séchage.

13. Procédé selon la revendication 12, caractérisé en ce que la dispersion de particules de polymère contient de 0,1 à 50 % en poids de particules de polymère.

14. Procédé selon la revendication 12 ou 13, caractérisé en ce que les quantités relatives de matrice et de dispersion aqueuse mises en oeuvre correspondent à un rapport pondéral matrice/particules de polymère de l'ordre de 0,01 à 1 000.

15. Support biologiquement actif formé du support pour molécules biologiquement actif faisant l'objet de l'une quelconque des revendications 1 à 11, dont le polymère est "sensibilisé" par des molécules biologiquement actives.

16. Support biologiquement actif selon la revendication 15, caractérisé en ce que les substances "sensibilisantes" biologiquement actives sont des proteines, des antigènes, des médicaments.

17. Support biologiquement actif selon la revendication 15 ou 16, caractérisé en ce que la quantité de substance "sensibilisante" du polymère représente de l'ordre de 0,01 à 15 % en poids par rapport au poids de particules de polymère.

18. Procédé de préparation du support biologiquement actif faisant l'objet de l'une quelconque des revendications 15 à 17 par fixation de molécules biologiquement actives sur le support faisant l'objet de l'une quelconque des revendications 1 à 11.

19. Procédé selon la revendication 18, caractérisé en ce que la quantité de molécules biologiquement actives mise en oeuvre est suffisante pour sensibiliser de 0,01 à 15 % du poids de polymère.

20. Utilisation du support faisant l'objet de l'une quelconque des revendications 1 à 11 et 15 à 17 en biologie.

21. Réactif de diagnostic constitué par le support biologiquement actif faisant l'objet de l'une quelconque des revendications 15 à 17.

## Revendications (Revendications pour l'(les) Etat(s) contractant(s) suivant(s) : suivant : ES)

1. Procédé de préparation d'un support pour molécules biologiquement actives constitué :
- d'une matrice inorganique, poreuse, non hydrosoluble,
- et à la surface de ladite matrice, de particules de polymère non hydrosoluble susceptibles de fixer des molécules biologiquement actives par dépôt de particules de polymère non hydrosoluble susceptibles de fixer des molécules biologiquement actives à la surface d'une matrice inorganique, poreuse, non hydrosoluble, par contact d'une dispersion aqueuse desdites particules de polymère avec la matrice poreuse et séchage.

2. Procédé selon la revendication 1, caractérisé en ce que ladite matrice présente un volume poreux de l'ordre de 5,5 à 1,8 ml/g, un diamètre de pores de l'ordre de 0,005 à 10 microns et une surface spécifique de l'ordre de 2 à 1 000_{m2/}g.

3. Procédé selon la revendication 1 ou 2, caractérisé en ce que ladite matrice se présente sous forme de particules de diamètre de l'ordre de 4 microns à 5 mm.

4. Procédé selon l'une quelconque des revendications précédentes, caractérisé en ce que la matière constituant la matrice est de la silice, de l'alumine, du verre poreux.

5. Procédé selon l'une quelconque des revendications précédentes, caractérisé en ce que les particules de polymère présentent une granulométrie de l'ordre de 0,05 à 20 microns.

6. Procédé selon la revendication 5, caractérisé en ce que le diamètre des particules de polymère est inférieur à celui des pores de la matrice.

7. Procède selon l'une quelconque des revendications précédentes, caractérisé en ce que le poids de la matrice correspond à environ 1 à 1 000 fois celui des particules de polymère.

8. Procédé selon l'une quelconque des revendications précédentes, caractérisé en ce que le polymère non hydrosoluble dérive d'un monomère vinylaromatique, d'un alkylester d'acide a-z insaturé, d'un ester d'un acide carboxylique insaturé, du chlorure de vinyle, du chlorure de vinylidène, d'un diène conjugué, d'un monomère insaturé présentant une fonction nitrile, d'un siloxane.

9. Procédé selon l'une quelconque des revendications précédentes, caractérisé en ce que le polymère non hydrosoluble dérive d'un monomère non miscible à l'eau et de moins de 10 % d'au moins un comonomère portant un groupe ionogène ou réactif.

10. Procédé selon la revendication 9, caractérisé en ce que le comonomère est du vinylbenzène sulfonate, un sulfoalkylester d'acide insaturé, un acide carboxylique insaturé, un hydroxyal- kylacrylate ou méthacrylate, un aminoalkyles- ter d'acide insaturé, l'acrylamide, le chlorure de vinylbenzène, le méthacrylate de glycidyle.

11. Procédé selon l'une quelconque des revendications précédentes, caractérisé en ce que la matrice présente en surface des groupes fonctionnels susceptibles de réagir par covalence avec les particules de polymère.

12. Procédé selon la revendication 11, caractérisé en ce que la dispersion de particules de polymère contient de 0,1 a 50 % en poids de particules de polymère.

13. Procédé selon la revendication 12, caractérisé en ce que les quantités relatives de matrice et de dispersion aqueuse mises en oeuvre correspondent à un rapport pondéral matrice/particules de polymère de l'ordre de 0,01 à 1000.

14. Procédé de préparation d'un support biologiquement actif formé du support pour molécules biologiquement actives tel que défini aux revendications 1 à 11, dont le polymère est "sensibilisé par des molécules biologiquement actives par fixation de molécules biologiquement actives sur ledit support.

15. Procédé biologiquement actif selon la revendication 14, caractérisé en ce que les substances "sensibilisantes" biologiquement actives sont des protéines, des antigènes, des médicaments.

16. Procédé, biologiquement actif selon la revendication 14 ou 15 , caractérisé en ce que la quantité de substance "sensibilisante" du polymère représente de l'ordre de 0,01 à 15 % en poids par rapport au poids de particules de polymère.

17. Procédé selon la revendication 14, caractérisé en ce que la quantité de molécules biologiquement actives mise en oeuvre est suffisante pour sensibiliser de 0,01 à 15 % en poids du polymère.

18. Utilisation du support décrit aux revendications 1 à 13 ou 14 à 17 en biologie.

## Claims (Claims for the following Contracting State(s) : AT, BE, CH, LI, DE, FR, GB, IT, LU, NL, SE)

1. Support for biologically active molecules, characterized in that it consists:
- of a porous, water-insoluble inorganic matrix, and
- on the surface of the said matrix, of water-insoluble polymer particles capable of fixing biologically active molecules.

2. Support according to Claim 1, characterized in that the said matrix has a pore volume of the order of 0.5 to 1.8 ml/g, a pore diameter of the order of 0.005 to 10 microns and a specific surface area of the order of 2 to 1,000 m²/g.

3. Support according to claim 1 or 2, characterized in that the said matrix is in the form of particles of diameter of the order of 4 microns to 5 mm.

4. Support according to any one of the preceding claims, characterized in that the material of which the matrix consists is silica, alumina or porous glass.

5. Support according to any one of the preceding claims, in which the polymer particles have a particle size of the order of 0.05 to 20 microns.

6. Support according to claim 5, in which the particle diameter of the polymer is smaller than that of the pores of the matrix.

7. Support according to any one of the preceding claims, in which the weight of the matrix corresponds to approximately 1 to 1,000 times that of the polymer particles.

8. Support according to any one of the preceding claims, in which the water-insoluble polymer is derived from a vinylaromatic monomer, an alkyl ester of an a,;8-unsaturated acid, an ester of an unsaturated carboxylic acid, vinyl chloride, vinylidene chloride, a conjugated diene, an unsaturated monomer containing a nitrile functional group, or a siloxane.

9. Support according to any one of the preceding claims, in which the water-insoluble polymer is derived from a water-immiscible monomer and from less than 10 % of at least one comonomer containing an ionizable or reactive group.

10. Support according to claim 9, in which the comonomer is vinylbenzene sulphonate, a sulphoalkyl ester of an unsaturated acid, an unsaturated carboxylic acid, a hydroxyalkyl acrylate or methacrylate, an aminoalkyl ester of an unsaturated acid, acrylamide, vinylbenzene chloride or glycidyl methacrylate.

11. Support according to any one of the preceding claims, in which the matrix surface has functional groups capable of reacting covalently with the polymer particles.

12. Process for the preparation of the support forming the subject of any one of the preceding claims by deposition of water-insoluble particles capable of fixing biologically active molecules on the surface of a porous water-insoluble inorganic matrix, by contacting of an aqueous dispersion of the said polymer particles with the porous matrix and drying.

13. Process according to claim 12, in which the dispersion of polymer particles contains from 0.1 to 50 % by weight of polymer particles.

14. Process according to claim 12 or 13, in which the relative quantities of matrix and aqueous dispersion which are used correspond to a matrix/polymer particles weight ratio of the order of 0.01 to 1000.

15. Biologically active support formed from the support for biologically active molecules forming the subject of any one of claims 1 to 11, in which the polymer is "sensitized" by biologically active molecules.

16. Biologically active support according to claim 15, in which the biologically active "sensitizing" substances are proteins, antigens or medications.

17. Biologically active support according to claim 15 or 16, in which the quantity of "sensitizing" substance of the polymer represents of the order of 0.01 to 15 % by weight relative to the weight of the polymer particles.

18. Process for the preparation of the biologically active support forming the subject of any one of claims 15 to 17 by fixing biologically active molecules onto the support forming the subject of any one of claims 1 to 11.

19. Process according to claim 18, in which the quantity of biologically active molecules which is used is sufficient to sensitize from 0.01 to 15 % of the weight of polymer.

20. Use of the support forming the subject of any one of claims 1 to 11 and 15 to 17 in biology.

21. Diagnostic reagent consisting of the biologically active support forming the subject of any one of claims 15 to 17.

## Claims (Claims for the following Contracting State(s) : ES)

1. Process for the preparation of a support for biologically active molecules, consisting:
- of a porous, water-insoluble inorganic matrix, and
- on the surface of the said matrix, of water-insoluble polymer particles capable of fixing biologically active molecules by deposition of particles of water-insoluble polymer capable of fixing biologically active molecules on the surface of a porous water-insoluble inorganic matrix, by contact of an aqueous dispersion of the said polymer particles with the porous matrix and drying.

2. Process according to Claim 1, characterized in that the said matrix has a pore volume of the order of 0.5 to 1.8 ml/g, a pore diameter of the order of 0.005 to 10 microns and a specific surface area of the order of 2 to 1000 m²/g.

3. Process according to Claim 1 or 2, characterized in that the said matrix is in the form of particles of diameter of the order of 4 microns to 5 mm.

4. Process according to any one of the preceding claims, characterized in that the materials of which the matrix consists is silica, alumina or porous glass.

5. Process according to any one of the preceding claims, characterized in that the polymer particles have a particle size of the order of 0.05 to 20 microns.

6. Process according to Claim 5, characterized in that the diameter of the polymer particles is smaller than that of the pores of the matrix.

7. Process according to any one of the preceding claims, characterized in that the weight of the matrix corresponds to approximately 1 to 1000 times that of the polymer particles.

8. Process according to any one of the preceding claims, characterized in that the water-insoluble polymer is derived from a vinylaromatic monomer, an alkyl ester of an a-z-unsaturated acid, an ester of an unsaturated carboxylic acid, vinyl chloride, vinylidene chloride, a conjugated diene, an unsaturated monomer containing a nitrile functional group or a siloxane.

9. Process according to any one of the preceding claims, characterized in that the water-insoluble polymer is derived from a water-immiscible monomer and from less than 10 % of at least one comonomer containing an ionizable or reactive group.

10. Process according to Claim 9, characterized in that the comonomer is vinylbenzene sulphonate, a sulphoalkyl ester of an unsaturated acid, an unsaturated carboxylic acid, a hydroxyalkyl acrylate or methacrylate, an aminoalkyl ester of an unsaturated acid, acrylamide, vinylbenzene chloride or glycidyl methacrylate.

11. Process according to any one of the preceding claims, characterized in that the matrix surface has functional groups capable of reacting covalently with the polymer particles.

12. Process according to Claim 11, characterized in that the dispersion of polymer particles contains from 0.1 to 50 % by weight of polymer particles.

13. Process according to Claim 12, characterized in that the relative quantities of matrix and of aqueous dispersion which are used correspond to a matrix/polymer particles weight ratio of the order of 0.01 to 1000.

14. Process for the preparation of a biologically active support formed from the support for biologically active molecules as defined in Claims 1 to 11, in which the polymer is "sensitized" by biologically active molecules by fixing biologically active molecules onto the said support.

15. Biologically active process according to Claim 15, characterized in that the biologically active "sensitizing" substances are proteins, antigens or medications.

16. Biologically active process according to Claim 14 or 15, characterized in that the quantity of "sensitizing" substance of the polymer represents of the order of 0.01 to 15 % by weight relative to the weight of the polymer particles.

17. Process according to Claim 14, characterized in that the quantity of biologically active molecules which is used is sufficient to sensitize from 0.01 to 15 % by weight of the polymer.

18. Use of the support described in Claims 1 to 13 or 14 to 17 in biology.

## Patentansprüche (Patentansprüche für folgende(n) Vertragsstaat(en) : AT, BE, CH, LI, DE, FR, GB, IT, LU, NL, SE)

1. Träger für biologisch aktive Moleküle, dadurch gekennzeichnet, daß er besteht aus:
- einer nicht-wasserlöslichen porösen anorganischen Matrix und
- nicht-wasserlöslichen Polymerteilchen, die biologisch aktive Moleküle fixieren können, auf der Oberfläche der genannten Matrix.

2. Träger nach Anspruch 1, dadurch gekennzeichnet, daß die genannte Matrix ein Porenvolumen in der Größenordnung von 0,5 bis 1,8 ml/g, einen Porendurchmesser in der Größenordnung von 0,005 bis 10 um und eine spezifische Oberflächengröße in der Größenordnung von 2 bis 1000 m²/g aufweist.

3. Träger nach Anspruch 1 oder 2, dadurch gekennzeichnet, daß die genannte Matrix in Form von Teilchen mit einem Durchmesser in der Größenordnung von 4 um bis 5 mm vorliegt.

4. Träger nach einem der vorhergehenden Ansprüche, dadurch gekennzeichnet, daß das die Matrix aufbauende Material Siliciumdioxid, Aluminiumoxid oder poröses Glas ist.

5. Träger nach einem der vorhergehenden Ansprüche, dadurch gekennzeichnet, daß die Polymerteilchen eine Granulometrie in der Größenordnung von 0,05 bis 20 um haben.

6. Träger nach Anspruch 5, dadurch gekennzeichnet, daß der Durchmesser der Polymerteilchen kleiner ist als derjenige der Poren der Matrix.

7. Träger nach einem der vorhergehenden Ansprüche, dadurch gekennzeichnet, daß das Gewicht der Matrix etwa dem 1-fachen bis 1000- fachen desjenigen der Polymerteilchen entspricht.

8. Träger nach einem der vorhergehenden Ansprüche, dadurch gekennzeichnet, daß das nicht-wasserlösliche Polymer abgeleitet ist von einem vinylaromatischen Monomer, einem Alkylester einer a,ß-ungesättigten Säure, einem Ester einer ungesättigten Carbonsäure, Vinylchlorid, Vinylidenchlorid, einem konjugierten Dien, einem ungesättigten Monomer, das eine Nitrilfunktion aufweist, und einem Siloxan.

9. Träger nach einem der vorhergehenden Ansprüche, dadurch gekennzeichnet, daß das nicht-wasserlösliche Polymer abgeleitet ist von einem mit Wasser nicht mischbaren Monomer und mindestens 10 % mindestens eines Comonomers, das eine ionogene oder reaktionsfähige Gruppe trägt.

10. Träger nach Anspruch 9, dadurch gekennzeichnet, daß das Comonomer Vinylbenzolsulfonat, ein Sulfoalkylester einer ungesättigten Säure, eine ungesättigte Carbonsäure, ein Hydroxyalkylacrylat oder -methacrylat, ein Aminoalkylester einer ungesättigten Säure, Acrylamid, Vinylbenzolchlorid und Glycidylmethacrylat ist.

11. Träger nach einem der vorhergehenden Ansprüche, dadurch gekennzeichnet, daß die Matrix an der Oberfläche funktionelle Gruppen aufweist, die mit den Polymerteilchen covalent reagieren können.

12. Verfahren zur Herstellung eines Trägers nach einem der vorhergehenden Ansprüche durch Abscheidung von nichtwasserlöslichen Polymerteilchen, die biologisch aktive Moleküle fixieren können, auf der Oberfläche einer nicht- wasserlöslichen, porösen anorganischen Matrix durch Inkontaktbringen einer wäßrigen Dispersion der genannten Polymerteilchen mit der porösen Matrix und Trocknen.

13. Verfahren nach Anspruch 12, dadurch gekennzeichnet, daß die Dispersion der Polymerteilchen 0,1 bis 50 Gew.-% Polymerteilchen enthält.

14. Verfahren nach Anspruch 12 oder 13, dadurch gekennzeichnet, daß die verwendeten relativen Mengen an Matrix und wäßriger Dispersion einem Gewichtsverhältnis Matrix/Polymerteilchen in der Größenordnung von 0,01 bis 1000 entsprechen.

15. Biologisch aktiver Träger, der aus dem Träger für biologisch aktive Moleküle nach einem der Ansprüche 1 bis 11 gebildet ist, dessen Polymer durch biologisch aktive Moleküle "sensibilisiert" worden ist.

16. Biologisch aktiver Träger nach Anspruch 15, dadurch gekennzeichnet, daß die "sensibilisierenden" biologisch aktiven Substanzen Proteine, Antigene und Arzneimittel sind.

17. Biologisch aktiver Träger nach Anspruch 15 oder 16, dadurch gekennzeichnet, daß die Menge der "sensibilisierenden" Substanz des Polymers in der Größenordnung von 0,01 bis 15 Gew.-%, bezogen auf das Gewicht der Polymerteilchen, liegt.

18. Verfahren zur Herstellung des biologisch aktiven Trägers nach einem der Ansprüche 15 bis 17 durch Fixierung von biologisch aktiven Molekülen an dem Träger nach einem der Ansprüche 1 bis 11.

19. Verfahren nach Anspruch 18, dadurch gekennzeichnet, daß die eingesetzte Menge an biologisch aktiven Molekülen ausreicht, um 0,01 bis 15 Gew.-% des Polymers zu sensibilisieren.

20. Verwendung des Trägers nach einem der Ansprüche 1 bis 11 und 15 bis 17 in der Biologie.

21. Diagnostisches Reagens, das besteht aus dem biologisch aktiven Träger nach einem der Ansprüche 15 bis 17.

## Patentansprüche (Patentansprüche für folgende(n) Vertragsstaat(en) : ES)

1. Verfahren zur Herstellung eines Trägers für biologisch aktive Moleküle, der besteht aus:
- einer nicht-wasserlöslichen porösen anorganischen Matrix und
- nicht-wasserlöslichen Polymerteilchen, die biologisch aktive Moleküle an der Oberfläche der genannten Matrix fixieren können,
durch Abscheidung von nicht-wasserlöslichen Polymerteilchen, die biologisch aktive Moleküle fixieren können, auf der Oberfläche einer nicht-wasserlöslichen, porösen anorganischen Matrix durch Kontaktieren einer wäßrigen Dispersion der genannten Polymerteilchen mit der porösen Matrix und Trocknen.

2. Verfahren nach Anspruch 1, dadurch gekennzeichnet, daß die genannte Matrix ein Porenvolumen in der Größenordnung von 0,5 bis 1,8 ml/g, einen Porendurchmesser in der Größenordnung von 0,005 bis 10 um und eine spezifische Oberflächengröße in der Größenordnung von 2 bis 1000 m²/g aufweist.

3. Verfahren nach Anspruch 1 oder 2, dadurch gekennzeichnet, daß die genannte Matrix in Form von Teilchen mit einem Durchmesser in der Größenordnung von 4 um bis 5 mm vorliegt.

4. Verfahren nach einem der vorhergehenden Ansprüche, dadurch gekennzeichnet, daß das die Matrix aufbauende Material Siliciumdioxid, Aluminiumoxid oder poröses Glas ist.

5. Verfahren nach einem vorhergehenden Ansprüche, dadurch gekennzeichnet, daß die Polymerteilchen eine Granulometrie in der Größenordnung von 0,05 bis 20 um haben.

6. Verfahren nach Anspruch 5, dadurch gekennzeichnet, daß der Durchmesser der Polymerteilchen kleiner ist als derjenige der Poren der Matrix.

7. Verfahren nach einem der vorhergehenden Ansprüche, dadurch gekennzeichnet, daß das Gewicht der Matrix etwa dem 1-fachen bis dem 1000-fachen desjenigen der Polymerteilchen entspricht.

8. Verfahren nach einem der vorhergehenden Ansprüche, dadurch gekennzeichnet, daß das nicht-wasserlösliche Polymer abgeleitet ist von einem vinylaromatischen Monomer, einem Alkylester einer a,ß-ungesättigten Säure, einem Ester einer ungesättigten Carbonsäure, Vinylchlorid, Vinylidenchlorid, einem konjugierten Dien, einem ungesättigten Monomer, das eine Nitrilfunktion aufweist, und einem Siloxan.

9. Verfahren nach einem der vorhergehenden Ansprüche, dadurch gekennzeichnet, daß das nicht-wasserlösliche Polymer abgeleitet ist von einem mit Wasser nicht mischbaren Monomer und mindestens 10 % mindestens eines Comonomers, das eine ionogene oder reaktionsfähige Gruppe trägt.

10. Verfahren nach Anspruch 9, dadurch gekennzeichnet, daß das Comonomer Vinylbenzolsulfonat, ein Sulfoalkylester einer ungesättigten Säure, eine ungesättigte Carbonsäure, ein Hydroxyalkylacrylat oder -methacrylat, ein Aminoalkylester einer ungesättigten Säure, Acrylamid, Vinylbenzolchlorid und Glycidylmethacrylat ist.

11. Verfahren nach einem der vorhergehenden Ansprüche, dadurch gekennzeichnet, daß die Matrix an der Oberfläche funktionelle Gruppen aufweist, die mit den Polymerteilchen kovalent reagieren können.

12. Verfahren nach Anspruch 11, dadurch gekennzeichnet, daß die Polymerteilchen-Dispersion 0,1 bis 50 Gew.-% Polymerteilchen enthält.

13. Verfahren nach Anspruch 12, dadurch gekennzeichnet, daß die verwendeten relativen Mengen an Matrix und wäßriger Dispersion einem Gewichtsverhältnis Matrix/Polymerteilchen in der Größenordnung von 0,01 bis 1000 entsprechen.

14. Verfahren zur Herstellung eines biologisch aktiven Trägers, der aus dem Träger für biologisch aktive Moleküle nach den Ansprüchen 1 bis 11 gebildet ist, dessen Polymer durch biologisch aktive Moleküle "sensibilisiert" worden ist durch Fixierung von biologisch aktiven Molekülen an dem genannten Träger.

15. Verfahren nach Anspruch 14, dadurch gekennzeichnet, daß die "sensibilisierenden" biologisch aktiven Substanzen Proteine, Antigene oder Arzneimittel sind.

16. Verfahren nach Anspruch 14 oder 15, dadurch gekennzeichnet, daß die Menge der "sensibilisierenden" Substanz des Polymers in der Größenordnung von 0,01 bis 15 Gew.-%, bezogen auf das Gewicht der Polymerteilchen, liegt.

17. Verfahren nach Anspruch 14, dadurch gekennzeichnet, daß die verwendete Menge der biologisch aktiven Moleküle ausreicht, um 0,01 bis 15 Gew.-% des Polymers zu sensibilisieren.

18. Verwendung des in den Ansprüchen 1 bis 13 oder 14 bis 17 beschriebenen Trägers in der Biologie.
